# EUROPEAN PATENT APPLICATION

(11) **EP 3 581 644 A1**
(43) Date of publication of application: **18.12.2019**
(21) Application number: 18751085.4
(22) Date of filing: 13.02.2018
(51) Int. Cl.: C12N 5/074, C12N 1/04

(54) **HUMAN STEM CELL PRESERVATIVE, HUMAN STEM CELL SUSPENSION, AND HUMAN STEM CELL PRESERVATION METHOD**

(30) Priority: 10.02.2017 JP 2017022739
(71) Applicant: FUJIFILM Corporation, Tokyo 106-8620 (JP); National University Corporation Tokyo Medical and Dental University, Bunkyo-ku Tokyo 113-8510 (JP)
(72) Inventor: YANADA Shinobu, Gamagori-shi Aichi 443-0022 (JP); HADA Satoko, Gamagori-shi Aichi 443-0022 (JP); SUZUKI Kento, Gamagori-shi Aichi 443-0022 (JP); SEKIYA Ichiro, Tokyo 113-8510 (JP); MIZUNO Mitsuru, Tokyo 113-8510 (JP); KATANO Hisako, Tokyo 113-8510 (JP); OZEKI Nobutake, Tokyo 113-8510 (JP); OTABE Kouji, Tokyo 113-8510 (JP)
(74) Representative: Hoffmann Eitle
(86) International application number: PCT/JP2018/004794
(87) International publication number: WO 2018/147457

(57) **Abstract**

An object of the present invention is to provide a preservative solution for human stem cells, a human stem cell suspension, and a method for preserving human stem cells, which enable preservation of human stem cells at a high survival rate for cells. According to the present invention, a preservative solution for human stem cells is provided. The preservative solution includes at least human serum, in which a volume ratio of human serum with respect to the entire preservative solution is 0.70 or more, and the human stem cells are preserved at a temperature higher than 0°C.

## Description

### BACKGROUND OF THE INVENTION

### 1. Field of the Invention

The present invention relates to a preservative solution for human stem cells, which contains human serum. The present invention further relates to a human stem cell suspension which contains human serum, and a method for preserving human stem cells in which human serum is used.

### 2. Description of the Related Art

In recent years, regenerative medicine in which living cells are transplanted to patients for performing therapies has been used actively. In order for cells to be transplanted to exhibit a therapeutic effect, a survival rate for cells is required to be maintained at a high level until immediately before the cells are used in transplantation. Accordingly, research on technology for preserving cells while maintaining cell activity is conducted.

In regenerative medicine, stem cells separated and collected from patient tissue are frequently used in autologous cell therapies in which cells of patients themselves are used. Except for some therapies in which stem cells are transplanted immediately after being collected from patients, expansion cultures are carried out at cell processing facilities or the like in many autologous cell therapies because it is necessary to secure the number of cells required for obtaining sufficient therapeutic effects. Thereafter, as soon as the number of cells required is obtained, the cells are transported to a medical institution for transplantation to a patient and are immediately transplanted to a patient upon arrival at the medical institution. Accordingly, stem cells are preserved only during a short period of time from transportation and arrival at a medical institution until the start of transplantation.

As a method for preserving cells for a short period of time, a method for preserving cells in a suspension state without freezing is known. For example, Patent Document 1 discloses a cell preservative solution for cryopreservation which contains at least saccharides, sodium ions, potassium ions, hydrogen carbonate ions and/or carbonate ions, and phosphate ions; which does not contain glycerol; and which defines a ratio of a molar concentration of sodium ions to a molar concentration of potassium ions, a content of hydrogen carbonate ions and/or carbonate ions, types of saccharides, and an osmotic pressure and pH.

### Prior Art Documents

### Patent Documents

Patent Document 1: JP4947948B

### SUMMARY OF THE INVENTION

As described above, in the method for preserving cells in a suspension state without freezing, a cell preservative solution including various components is used. In a case where cells preserved in a preservative solution is transplanted to a patient, the above-described components are administered together with the cells into the body of a patient. Therefore, it is necessary to select components that do not exhibit toxicity or antigenicity.

In addition, in a case of transplantation at a medical institution, cells are handled in a clean environment such as an operating room. Therefore, it is important to reduce the number of operations before transplantation as much as possible to maintain cleanliness. Accordingly, a case in which transplantation can be performed without removing a cell preservative solution, can contribute to maintaining a high level of cleanliness.

An object of the present invention is to provide a preservative solution for human stem cells, a human stem cell suspension, and a method for preserving human stem cells, which enable preservation of human stem cells at a high survival rate for cells. Another object of the present invention is to provide a preservative solution for human stem cells, a human stem cell suspension, and a method for preserving human stem cells, which enable safe transplantation of human stem cells even in a case where a preservative solution is present.

As a result of intensive studies to achieve the above-mentioned objects, the inventors of the present invention have found that human stem cells can be preserved at a high survival rate for cells by using, as a preservative solution for human stem cells, a preservative solution which contains at least human serum and in which a volume ratio of human serum with respect to the entire preservative solution is 0.70 or more. The present invention has been completed based on these findings.

In other words, according to the present invention, the following invention is provided.
(1) A preservative solution for human stem cells, comprising at least human serum, in which a volume ratio of human serum with respect to the entire preservative solution is 0.70 or more, and the human stem cells are preserved at a temperature higher than 0°C.
(2) The preservative solution according to (1), in which the human serum is human serum obtained by a blood separation operation that uses beads coated with a blood clot promoting enzyme.
(3) The preservative solution according to (1) or (2), further comprising at least one or more selected from the group consisting of Ringer's solution listed in the Japanese Pharmacopoeia, Dulbecco's modified Eagle's medium, alpha-minimum essential medium, and alpha-modified Eagle's medium.
(4) The preservative solution according to any one of (1) to (3), in which the human stem cells are human stem cells derived from synovial membranes.
(5) A human stem cell suspension comprising the preservative solution according to any one of (1) to (4), and human stem cells suspended in the preservative solution.
(6) The human stem cell suspension according to (5), in which the human stem cell suspension is directly administered to a patient.
(7) A method for preserving human stem cells, comprising preserving a human stem cell suspension obtained by suspending human stem cells in the preservative solution according to any one of (1) to (4).
(8) The method according to (7), in which the human stem cell suspension is preserved at a temperature of 4°C to 20°C.

According to a preservative solution, a stem cell suspension, and a preservation method of the present invention, human stem cells can be preserved at a high survival rate for cells. Furthermore, according to the present invention, the human stem cell suspension can be administered to a patient without an operation of removing the preservative solution for human stem cells.

### BRIEF DESCRIPTION OF THE DRAWINGS

- Fig. 1: shows measurement results of survival rates for cells.
- Fig. 2: shows images based on microscopic observation.
- Fig. 3: shows measurement results of survival rates for cells.
- Fig. 4: shows crystal violet stain images.
- Fig. 5: shows appearance of cartilage pellets.
- Fig. 6: shows histological evaluation of the cartilage pellets.

### DESCRIPTION OF THE PREFERRED EMBODIMENTS

Hereinafter, embodiments of the present invention will be described in detail.

A preservative solution for human stem cells of the embodiment of the present invention contains at least human serum, in which a volume ratio of human serum with respect to the entire preservative solution is 0.70 or more, and the human stem cells are preserved at a temperature higher than 0°C.

It is known to add serum (of which fetal bovine serum is frequently used) to a cell preservative solution for freezing preservation of cells. On the other hand, the present invention indicates that human stem cells can be preserved at a high survival rate for cells and a differentiation ability of human stem cells can be maintained, by using not fetal bovine serum but human serum as a preservative solution used for preserving human stem cells under a condition in which human stem cells are preserved at a temperature higher than 0°C (that is, a condition in which cells are not frozen).

It is also known to culture cells by using a medium into which serum is added. During the age when cell culture began, because the same kind of serum as cells was perceived to be favorable, human serum was added to a medium of human cells. However, thereafter, because of facts that fetal bovine serum shows better proliferation, human serum is used less as a component of a medium (Understandable Cell Culture with Q & A, p. 92, 2004, Yodosha Co., Ltd.). In addition, serum (human serum, bovine serum, and the like) contains various cell proliferation-promoting substances, cell damage-protecting factors, nutritional factors, and the like. However, in addition to the above-mentioned substances and factors, it is known that serum also contains cell proliferation inhibitory factors, differentiation promoting factors, complements, and the like, which are factors that cause damage to cells (Understandable Cell Culture with Q & A, p. 88 and p. 90, 2004, Yodosha Co., Ltd.). Based on the above description, findings are absolutely unexpected effects, in which human stem cells can be preserved at a high survival rate for cells and a differentiation ability of human stem cells can be maintained in a case where human stem cells are preserved by using a preservative solution which contains human serum and in which a volume ratio of human serum with respect to the entire preservative solution is high (0.70 or more).

In the field of regenerative medicine, the preservative solution for human stem cells of the embodiment of the present invention can be utilized as a cell preservative solution for maintaining a survival rate for stem cells in a case where stem cells are preserved only during a short period of time, from transportation from a cell processing facility to a medical institution where transplantation is performed and arrival at the medical institution, until the start of transplantation. In addition, according to the preservative solution for human stem cells of the embodiment of the present invention, it is not required to perform an operation of removing a cell preservative solution in a case of transplanting cells, and therefore transplantation can be performed even in a case where the cell preservative solution is present.

Stem cells are cells that have an ability to be divided to produce the same cells as themselves (an ability to self-renew) and an ability to differentiate into other types of cells, and are cells that can proliferate unlimitedly.

Examples of stem cells include pluripotent stem cells (having pluripotency) and multipotent stem cells (having multipotency).

The term "pluripotency" refers to an ability capable of differentiating into all of cell lines of the three germ layers (endoderm, mesoderm, and ectoderm) while an individual is not formed. Examples of pluripotent stem cells include embryonic stem cells (ES cells), embryonic germ cells (EG cells), nuclear transfer embryonic stem cells (ntES cell, also called ES cells derived from somatic cells), induced pluripotent stem cells (iPS cells), and the like.

The term "multipotency" refers to an ability capable of differentiating into various cell types, although cell lines into which cells can differentiate are limited. Examples of multipotent stem cells include hematopoietic stem cells, mesenchymal stem cells, hepatic stem cells, pancreatic stem cells, skin stem cells, and the like. It is known that mesenchymal stem cells can be obtained from various tissues such as bone marrow, fatty tissue, dental pulp, placental tissue, umbilical cord tissue, and synovial membranes.

As human stem cells, mesenchymal stem cells are preferable.

Examples of human stem cells include human stem cells derived from bone marrow, human stem cells derived from fat, human stem cells derived from dental pulp, human stem cells derived from placenta, human stem cells derived from umbilical cord, and human stem cells derived from synovial membranes. Human stem cells derived from synovial membranes are particularly preferable.

Serum is a pale yellow liquid component that can be made into a supernatant in a case where blood clots. In other words, serum is obtained by removing clotting factors after blood (whole blood) clots. Blood plasma contains clotting components, but serum does not contain clotting components or contains only small amounts thereof.

As human serum, for example, it is possible to use human serum obtained by a blood separation operation that uses beads coated with a blood clot promoting enzyme. A method for collecting human serum is not particularly limited.

Examples of blood separation operations include a series of operations in which fresh blood collected from a human is shaken at room temperature for 30 to 60 minutes while coming into contact with beads coated with a blood clot promoting enzyme, and then the shaken blood is separated into blood clot and serum by centrifugation to recover serum. As a means for preparing human serum, it is possible to use serum separated and prepared, from blood collected from a human, by using a blood component separation bag dedicated to serum preparation (CELLAID (registered trademark), JMS Co., Ltd.), but the means for preparing human serum is not limited thereto.

In the preservative solution of the embodiment of the present invention, a volume ratio of human serum with respect to the entire preservative solution is 0.70 or more. By setting a volume ratio of human serum with respect to the entire preservative solution as described above, a favorable survival rate for cells can be achieved.

The preservative solution of the embodiment of the present invention is a preservative solution for preserving human stem cells at a temperature higher than 0°C. In other words, the preservative solution of the embodiment of the present invention is a preservative solution for preserving human stem cells without freezing. A temperature for preservation is not particularly limited as long as a temperature is higher than 0°C, but an upper limit of the temperature is generally 50°C or lower. A lower limit of the temperature is generally 1°C or higher. Among the above range, a temperature for preservation is preferably 4°C to 20°C, and is more preferably 4°C to 15°C. In the present invention, by preserving human stem cells at a temperature higher than 0°C, a favorable survival rate for cells can be achieved.

In addition to human serum, the preservative solution of the embodiment of the present invention may further contain at least one or more selected from the group consisting of Ringer's solution listed in the Japanese Pharmacopoeia, Dulbecco's modified Eagle's medium, alpha-minimum essential medium, and alpha-modified Eagle's medium.

The preservative solution of the embodiment of the present invention can further contain, for example, antibiotics, antibacterial agents, antioxidants, vitamins, proteins, amino acids, pH indicators, chelating agents, and the like, as appropriate.

According to the present invention, there is provided a human stem cell suspension which contains the above-described preservative solution of the embodiment of the present invention, and contains human stem cells suspended in the above-mentioned preservative solution.

A method for suspending human stem cells in a preservative solution is not particularly limited, and can be performed by a general method. For example, it is possible to obtain a human stem cell suspension by mixing and stirring human stem cells and a preservative solution.

The human stem cell suspension of the embodiment of the present invention can be directly administered to a patient in a case where the human stem cell suspension is used for medical applications such as regenerative medicine. One of the advantages of the present invention is that the human stem cell suspension of the embodiment of the present invention can be administered to a patient without removing a preservative solution.

A concentration of cells in the human stem cell suspension of the embodiment of the present invention can be appropriately set according to conditions such as the types of cells, use of cells, size of cells, and preservation period, and is not particularly limited. For example, a concentration thereof is about 1.0 × 10⁴ to 1.0 × 10¹⁰ cells/mL, and is preferably about 1.0 × 10⁵ to 1.0 × 10⁹ cells/mL. In addition, human stem cells in a case of being suspended in a preservative solution may be passaged cells or non-passaged cells. In the case of passaged cells, the number of passage is not particularly limited, and is, for example, one time (passage 1) to nine times (passage 9), is preferably one time (passage 1) to five times (passage 5), and is more preferably one time (passage 1) to three times (passage 3).

The present invention also relates to a method for preserving human stem cells in which the preservative solution of the embodiment of the present invention is used. In other words, according to the present invention, there is further provided a method for preserving human stem cells, which includes preserving a human stem cell suspension obtained by suspending human stem cells in the preservative solution of the embodiment of the present invention.

As a method for preservation, a human stem cell suspension can be preserved under conditions in which the human stem cell suspension is not frozen. A temperature for preservation is as described above in the present specification, but is not particularly limited as long as a temperature is higher than 0°C. An upper limit of the temperature is generally 50°C or lower, preferably 45°C or lower, more preferably 40°C or lower, even more preferably 37°C or lower, and still even more preferably 20°C or lower, and particularly preferably 15°C or lower. A lower limit of the temperature is generally 1°C or higher, preferably 2°C or higher, and more preferably 4°C or higher. Among the above range, a temperature for preservation is preferably 4°C to 20°C, and is more preferably 4°C to 15°C.

In the present invention, a preservation period of human stem cells is not particularly limited, but is generally, for example, 5 minutes to 14 days.

In addition, a preservation container can be appropriately selected and used in consideration of the types of cells, preservation temperature, preservation period, use of cells after preservation, and the like. As a preservation container, it is possible to use, for example, a tube, a flask, an infusion bag, a cell culture bag, a syringe, or the like.

Human stem cells preserved by the preservation method of the embodiment of the present invention preferably have a high survival rate, a colony-forming ability, and a differentiation ability after preservation.

A method for measuring a survival rate for cells is not particularly limited. For example, it is possible to evaluate a survival rate for cells by using live/dead (registered trademark) assay kit (Logos Biosystems) and determining the number of living cells according to the procedure described in the instruction manual.

In a case of preservation for 48 hours in a thermostat set at 4°C by the preservation method of the embodiment of the present invention, a survival rate for cells is preferably 60% or more.

In a case of preservation for 48 hours in a thermostat set at 13°C by the preservation method of the embodiment of the present invention, a survival rate for cells is preferably 60% or more.

In a case of preservation for 48 hours in a thermostat set at 37°C by the preservation method of the embodiment of the present invention, a survival rate for cells is preferably 40% or more.

A method for evaluating whether or not human stem cells have a colony-forming ability is not particularly limited, and evaluation can be performed by a general method. It is possible to evaluate a colony-forming ability by, for example, seeding cells after preservation in culture dishes; culturing the cells at 37°C in 5% CO₂ atmosphere in an appropriate medium (for example, α-minimum essential medium that contains antibiotics and fetal bovine serum) for a predetermined period (for example, after 14 days); staining the cultured cells with 1% crystal violet solution; and observing whether or not a colony is formed.

A method for evaluating whether or not human stem cells have a differentiation ability is not particularly limited, and evaluation can be performed by a general method. For example, cells after preservation are transferred to a tube and are cultured in a cartilage differentiation medium that contains Transforming growth factor-β3 (TGF-β3) (final concentration: 10 ng/ml, Miltenyi Biotec K.K.) and Bone Morphogenetic Protein-2 (BMP2) (final concentration: 1 µg/ml, Medtronic). The medium is exchanged every 3 to 4 days. Histological evaluation is performed by observing toluidine blue stain images of cartilage pellets 21 days after the start of culture, and therefore a cartilage differentiation ability can be evaluated.

Hereinafter, the present invention will be specifically described based on examples, but the present invention is not limited to the scope of the examples.

### Examples

### <Collection of human serum>

Fresh blood was collected from three healthy volunteers, and serum was separated and recovered by using a blood component separation bag (CELLAID (registered trademark), JMS Co., Ltd.). Specifically, the collected fresh blood was shaken at room temperature for 30 minutes while coming into contact with beads coated with a blood clot promoting enzyme, and then the shaken blood was separated into blood clot and serum by centrifugation. The separated serum was filtered through a 0.45 µm nylon filter (Thermo Fisher Scientific), and then transferred to another preservation bag, and then preserved at -20°C until use.

For a method for using CELLAID (registered trademark), the attached document (first edition, attached document management number 12890Z01) issued by the manufacturer was referred to.

### <Preparation of human stem cells derived from synovial membranes>

Human synovial membrane tissue was collected from 10 donors. The collected synovial membrane tissue was immersed in a 3 mg/mL collagenase solution (Sigma-Aldrich Co. LLC.) and digested at 37°C for 3 hours. Thereafter, the solution after digestion reaction was passed through a cell strainer (pore diameter of 70 µm, Greiner Bio-One) to obtain synovial membrane cells. The synovial membrane cells obtained were cultured at 37°C in 5% CO₂ atmosphere in an α-minimum essential medium (Thermo Fisher Scientific) which contains antibiotic-antimycotic (final concentration of 1%, Thermo Fisher Scientific) and fetal bovine serum (final concentration of 10%). The number of cells was counted by Luna-FL (trade name) (Logos Biosystems).

### Test Example 1: Confirmation of influence of preservative solution on survival rate for cells

The human stem cells derived from synovial membranes (passage 2) which was obtained by the method described in <Preparation of human stem cells derived from synovial membranes> were recovered by using TrypLE (trade name) Select (Thermo Fisher Scientific). 2 × 10⁶ of the human stem cells derived from synovial membranes were suspended in 100 µl of human serum obtained by the method described in <Collection of human serum> (preservative solution 1) and 100µl of glucose-acetate Ringer's solution (KYOWA CritiCare) (preservative solution 2). The cell suspension was put into a preservation tube (Sumitomo Bakelite Co., Ltd.) to be preserved.

In order to confirm the influence of the preservative solution 1 and the preservative solution 2 on a survival rate for cells, the cell suspension preserved in each of the preservative solutions was preserved for 48 hours in a thermostat which was set at 4°C, 13°C, and 37°C. Survival rates (%) for cells before and after preservation were obtained by using live/dead (registered trademark) assay kit (Logos Biosystems) and determining the number of living cells according to the procedure described in the instruction manual. In addition, the cell morphology after preservation was observed with a microscope.

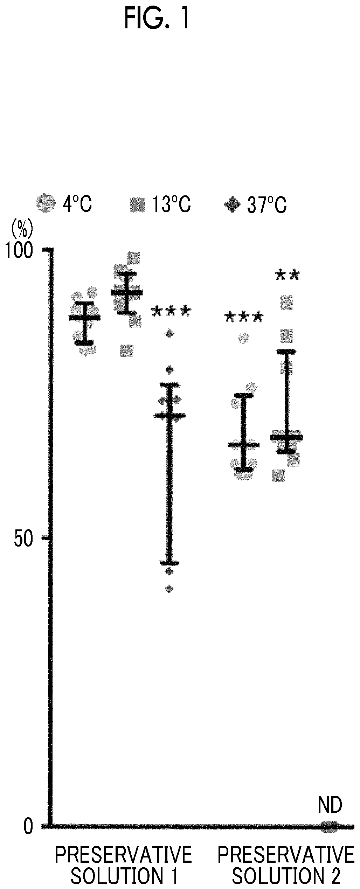

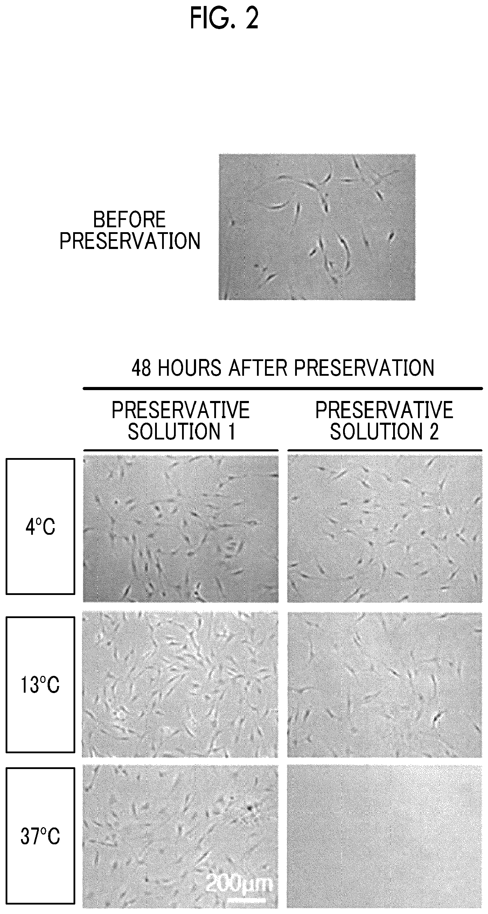

The results are shown in Fig. 1 (graph) and Fig. 2 (photographs).

As shown in Fig. 1, in conditions of any of temperatures 4°C, 13°C, and 37°C, survival rates for cells preserved in the human serum of the preservative solution 1 were higher than those of Ringer's solution not containing the human serum of the preservative solution 2. In particular, a significant difference was observed at 37°C.

As shown in Fig. 2, as a result of observing the morphology of the cells before and after preservation, no change was observed in the morphology in any case. In addition, as in the results of Fig. 1, no living cells were observed in a case of preservation at 37°C for 48 hours using the preservative solution 2.

### Test Example 2: Confirmation of influence of preservative solution using bovine serum

The human stem cells derived from synovial membranes (passage 2) which was obtained by the method described in <Preparation of human stem cells derived from synovial membranes> were recovered by using TrypLE (trade name) Select (Thermo Fisher Scientific). 1 × 10⁵ of the human stem cells derived from synovial membranes were suspended in 500 µl of α-minimum essential medium (Gibco) (preservative solution 3) and 500µl of fetal bovine serum (preservative solution 4). The cell suspension was put into a preservation tube (Sumitomo Bakelite Co., Ltd.) to be preserved.

In order to confirm the influence of the preservative solution 3 and the preservative solution 4 on a survival rate for cells, the cell suspension preserved in each of the preservative solutions was preserved for 3 days in a thermostat which was set at 18°C. Survival rates (%) for cells before and after preservation were obtained by using live/dead (registered trademark) assay kit (Logos Biosystems) and determining the number of living cells according to the procedure described in the instruction manual.

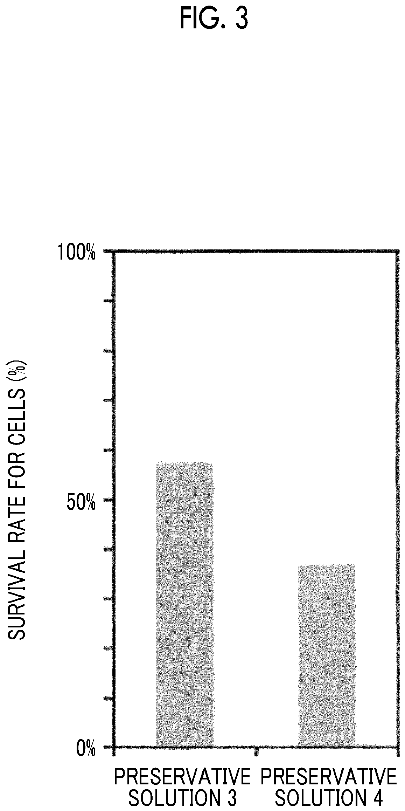

The results are shown in Fig. 3 (graph).

The preservative solution 3 showed almost the same result as the survival rate for cells after preservation at 13°C for 48 hours of the preservative solution 2 (Fig. 1). On the other hand, a survival rate of the cells preserved in the fetal bovine serum of the preservative solution 4 was a value lower than that of the preservative solution 3.

The results of Test Example 1 and Test Example 2 show that it is important that serum used for a cell preservative solution is human serum.

### Test Example 3: Confirmation of influence of preservative solution on colony-forming ability

The human stem cells derived from synovial membranes (passage 2) which was obtained by the method described in <Preparation of human stem cells derived from synovial membranes> were recovered by using TrypLE (trade name) Select (Thermo Fisher Scientific). 2 × 10⁶ of the human stem cells derived from synovial membranes were suspended in 100 µl of human serum obtained by the method described in <Collection of human serum> (preservative solution 1) and 100µl of glucose-acetate Ringer's solution (KYOWA CritiCare) (preservative solution 2). The cell suspension was put into a preservation tube (Sumitomo Bakelite Co., Ltd.) to be preserved.

In order to confirm the influence of the preservative solution 1 and the preservative solution 2 on a colony-forming ability, the cell suspension preserved in each of the preservative solutions was preserved for 48 hours in a thermostat which was set at 4°C, 13°C, and 37°C. 1 × 10⁴ cells after preservation for 48 hours were seeded in a 60 cm² culture dish, and were cultured at 37°C in 5% CO₂ atmosphere in an α-minimum essential medium (Thermo Fisher Scientific) which contains antibiotic-antimycotic (final concentration of 1%, Thermo Fisher Scientific) and fetal bovine serum (final concentration of 10%). 14 days after seeding, staining was performed with a 1% crystal violet solution, and colonies formed were observed.

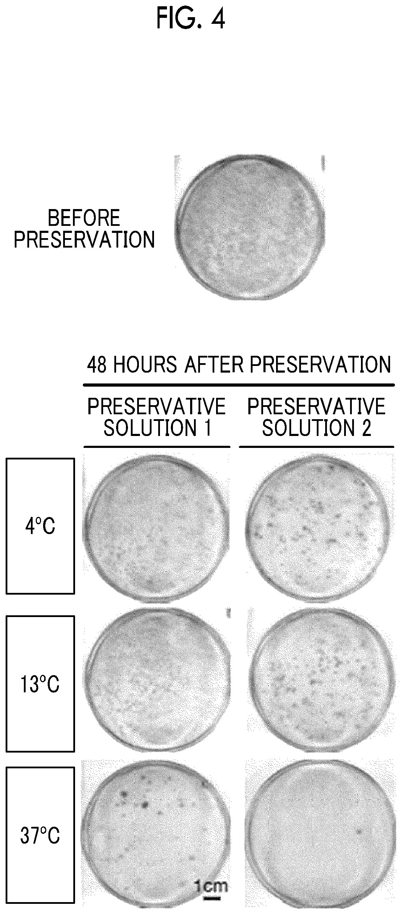

Fig. 4 shows crystal violet stain images.

Colony formation was observed in all cells of a group preserved in the human serum of the preservative solution 1. In a group of the preservative solution 2, no colonies were observed in cells after preservation at 37°C for 48 hours.

### Test Example 4: Confirmation of influence of preservative solution on cartilage differentiation ability

The human stem cells derived from synovial membranes (passage 2) which was obtained by the method described in <Preparation of human stem cells derived from synovial membranes> were recovered by using TrypLE (trade name) Select (Thermo Fisher Scientific). 2 × 10⁶ of the human stem cells derived from synovial membranes were suspended in 100 µl of human serum obtained by the method described in <Collection of human serum> (preservative solution 1) and 100µl of glucose-acetate Ringer's solution (KYOWA CritiCare) (preservative solution 2). The cell suspension was put into a preservation tube (Sumitomo Bakelite Co., Ltd.) to be preserved.

In order to confirm the influence of the preservative solution 1 and the preservative solution 2 on a cartilage differentiation ability, the cell suspension preserved in each of the preservative solutions was preserved for 48 hours in a thermostat which was set at 4°C, 13°C, and 37°C.

2.5 × 10⁵ cells after 48 hours of preservation were transferred to a 15 ml tube (BD) and were cultured in a cartilage differentiation medium that contains Transforming growth factor-β3 (TGF-β3) (final concentration: 10 ng/ml, Miltenyi Biotec K.K.) and Bone Morphogenetic Protein-2 (BMP2) (final concentration: 1 µg/ml, Medtronic). The medium was exchanged every 3 to 4 days. Histological evaluation was performed by observing toluidine blue stain images of cartilage pellets 21 days after the start of culture.

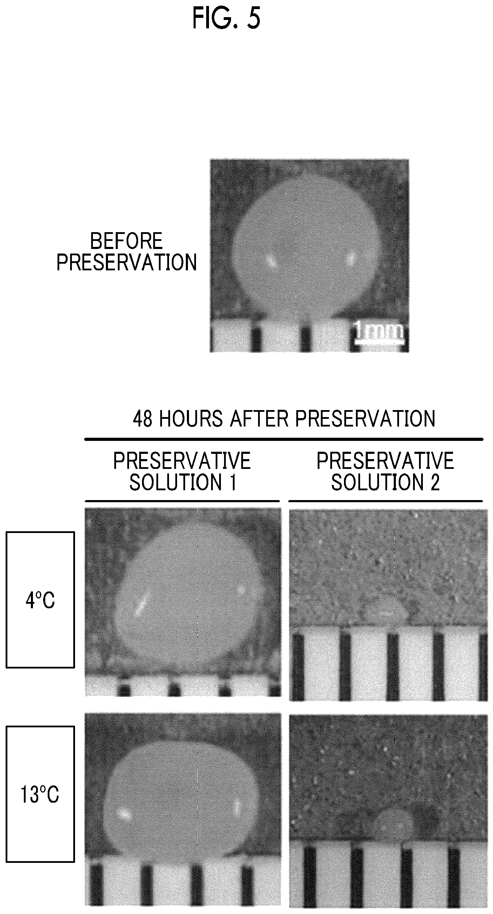

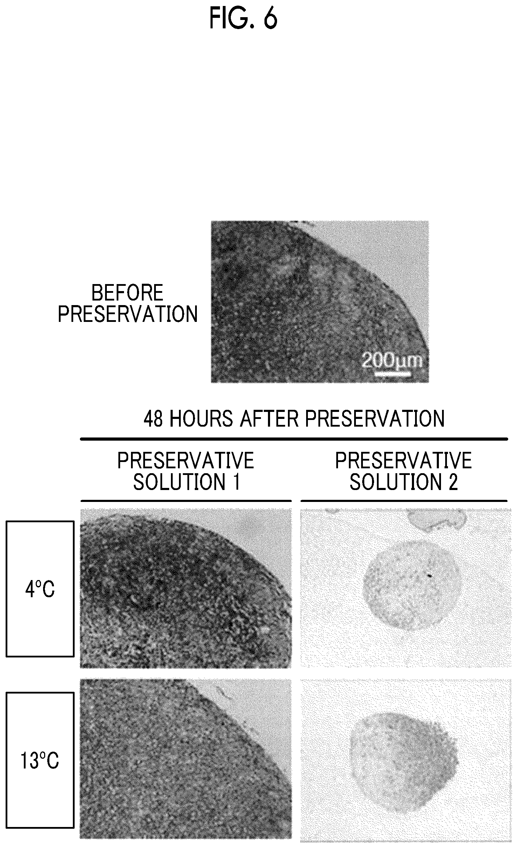

The results are shown in Fig. 5 (appearance) and Fig. 6 (images based on histological observation).

As shown in Fig. 5, a significant difference was observed in size of cartilage pellets produced from cells preserved in the human serum of the preservative solution 1, as compared to that of the Ringer's solution which does not contain the human serum of the preservative solution 2. In addition, the size of the cartilage pellets produced from cells preserved in the human serum of the preservative solution 1 was the same as that of cartilage pellets produced from cells before preservation. Based on this findings, it was shown that the cartilage differentiation ability of the human stem cells derived from synovial membranes was maintained in the case of the preservation using the preservative solution 1. On the other hand, in the case of the preservation at 37°C, formation of cartilage pellets was not observed regardless of the types of preservative solutions.

As shown in Fig. 6, a significant difference was observed in stain images of cartilage pellets produced from cells preserved in the human serum of the preservative solution 1, as compared to that of the Ringer's solution which does not contain the human serum of the preservative solution 2. Toluidine blue staining is a method for evaluating a degree of matrix produced by a cartilage pellet. These results show that, in addition to a cartilage formation ability, the cells preserved in the preservative solution 1 maintained a matrix production ability.

## Claims

1. A preservative solution for human stem cells, comprising:
at least human serum,
wherein a volume ratio of human serum with respect to the entire preservative solution is 0.70 or more, and
the human stem cells are preserved at a temperature higher than 0°C.

2. The preservative solution according to claim 1, wherein the human serum is human serum obtained by a blood separation operation that uses beads coated with a blood clot promoting enzyme.

3. The preservative solution according to claim 1 or 2, further comprising at least one or more selected from the group consisting of Ringer's solution listed in the Japanese Pharmacopoeia, Dulbecco's modified Eagle's medium, alpha-minimum essential medium, and alpha-modified Eagle's medium.

4. The preservative solution according to any one of claims 1 to 3, wherein the human stem cells are human stem cells derived from synovial membranes.

5. A human stem cell suspension comprising:
the preservative solution according to any one of claims 1 to 4; and
human stem cells suspended in the preservative solution.

6. The human stem cell suspension according to claim 5, wherein the human stem cell suspension is directly administered to a patient.

7. A method for preserving human stem cells, comprising preserving a human stem cell suspension obtained by suspending human stem cells in the preservative solution according to any one of claims 1 to 4.

8. The method according to claim 7, wherein the human stem cell suspension is preserved at a temperature of 4°C to 20°C.
